# EUROPEAN PATENT SPECIFICATION

(11) **EP 2 714 156 B1**
(45) Date of publication and mention of the grant of the patent: **11.07.2018**
(21) Application number: 12751148.3
(22) Date of filing: 24.05.2012
(51) Int. Cl.: A61M 5/32

(54) **NEEDLE-PROTECTION DEVICE FOR A SYRINGE**
NADELSCHUTZVORRICHTUNG FÜR EINE SPRITZE
DISPOSITIF DE PROTECTION D'AIGUILLE POUR UNE SERINGUE

(30) Priority: 02.06.2011 US 201161492567 P
(43) Date of publication of application: 09.04.2014
(73) Proprietor: West Pharmaceutical Services, Inc., Lionville, PA 19341-0645 (US)
(72) Inventor: BOYD, Robert, R., Athens, TX 75752 (US); DAVIS, Tommy, Athens, TX 75751 (US); CROW, Doug, Owen, Brownsboro, TX 75756 (US); GILLESPIE, Richard, David, Athens, TX 75752 (US)
(74) Representative: Lippert Stachow Patentanwälte Rechtsanwälte
(86) International application number: PCT/US2012/039385
(87) International publication number: WO 2012/166527

(56) References cited:
- WO-A1-2004/087242
- US-A- 4 911 693
- US-A- 4 923 447
- US-B1- 7 300 421
- US-B2- 7 875 006

## Description

### CROSS-REFERENCE TO RELATED APPLICATIONS

The present application claims priority to U.S. Provisional Patent Application No. 61/492,567, filed June 2, 2011 and entitled "Improved Syringe Safety System Activation Mechanism."

### BACKGROUND OF THE INVENTION

The present invention relates generally to a syringe safety mechanism and, more particularly, to a device that simplifies and improves the safety and reliability of a spring-loaded syringe device. The present invention relates in particular to the field of single-use pre-filled injection syringes, which are intended in particular for intramuscular or subcutaneous injections.

In order to ensure a higher level of safety for syringe devices, syringes have been provided with resilient means for displacing a protector around the needle of the syringe. The protector is intended to limit to the greatest possible extent the risk of accidental pricks after the injection to the user or patient. Once the contents of the syringe have been injected, the needle of the syringe is typically withdrawn from the patient and the protector is brought into a deployed position around the needle.

One such prior art device is disclosed in U.S. Patent No. 5,591,138 (Vaillancourt), which discloses a syringe device with resilient means. The device of Vaillancourt can be used in one of the following two manners: once the injection has been carried out with one hand, the user operates the unlocking means with his/her other hand in order to disengage a compression spring and to allow the deployment of the protector around the needle. Alternatively, the needle is introduced to a sufficient depth into the skin of the patient to bring about the unlocking of the spring. The spring is not able to relax when the protector is pressed against the skin of the patient. Instead, the spring relaxes when the entire syringe device is moved away from the patient.

The Vaillancourt device requires either manipulation with two hands or the protector to be pressed against the skin of the patient. This manipulation ensures neither a high level of ease of use for the practitioner, nor great comfort for the patient. Further, once the spring is unlocked, the decompression energy of the spring is often violently released without any control by the user being possible, which brings about a degree of instability of the device in the hand of the user. In addition, the rigid locking of the protector in the deployed position in order to prevent any re-use of the device, is possible only by the device being manipulated with two hands.

Another prior art syringe device that includes resilient means is disclosed in U.S. Patent No. 7,875,006 (Pessin), which is herein incorporated by reference in its entirety. While the device of Pessin can be quite effective and useful, especially for intramuscular or subcutaneous injections, this type of syringe device can be adversely affected by the torsion loading that occurs upon loading of the coil spring therein. The presence of torsion loading may impair the actuation process.

It has heretofore not been determined how to reduce the impact of the torsion loading in a simple and reliable way. The present invention accomplishes this objective.

### BRIEF SUMMARY OF THE INVENTION

Briefly stated, one aspect of the present invention is directed to a needle-protection device for a syringe including a protector support having a main portion and a secondary portion. The main portion has a generally tubular shape and including at least one recess extending therethrough and at least one rib extending across at least a portion of the recess. The rib is generally centrally positioned within the recess. The secondary portion includes at least one projection or groove capable of fixing the protector support to a neck of the syringe. A generally tubular needle protector has an inner diameter of the needle protector is at least slightly greater than an outer diameter of the protector support such that at least a portion of the protector support can be inserted within at least a portion of the needle protector. At least one hook extends radially inwardly from the needle protector. The needle protector is rotatable with respect to the protector support such that the at least one hook snaps over the at least one rib and locks the rotational position of the protector support with respect to the needle protector.

### BRIEF DESCRIPTION OF THE SEVERAL VIEWS OF THE DRAWINGS

The foregoing summary, as well as the following detailed description of the invention, will be better understood when read in conjunction with the appended drawings. For the purpose of illustrating the invention, there are shown in the drawings two embodiments which are presently preferred. It should be understood, however, that the invention is not limited to the precise arrangements and instrumentalities shown. In the drawings:
Fig. 1 is a partially exploded perspective view of an injection device according to a first preferred embodiment of the present invention, wherein the injection device is shown in a pre-assembly configuration
Fig. 2 is a front elevational view thereof in a retracted position;
Fig. 3 is a cross-sectional elevational view thereof taken from plane III-III of Fig. 2;
Fig. 4 is a top plan view thereof according to arrow IV indicated in Fig. 2;
Fig. 5 is a cross-sectional elevational view thereof taken from plane V-V in Fig. 4;
Fig. 6A is a cross-sectional elevational view of the injection device before use;
Fig. 6B is a top plan view thereof according to arrow VI indicated in Fig. 6A;
Fig. 7 is a cross-sectional elevational view of the injection device in use;
Fig. 8 is a cross-sectional elevational view of the injection device after use;
Fig. 9 is a cross-sectional elevational view of the injection device, wherein a syringe of the injection device is omitted for clarity;
Fig 10 is a cross-sectional elevational view taken from plane X-X in Fig. 8;
Fig. 11 is a cross-sectional elevational view of the injection device in a deployed position;
Fig. 12 is a cross-sectional view thereof taken from plane XII-XII in Fig. 11;
Fig. 13 is a cross-sectional elevational view of the injection device in a deployed position, wherein the syringe is omitted for clarity;
Fig. 14 is an enlarged top perspective view of a protector support of an injection device according to a second preferred embodiment of the present invention;
Fig. 15 is an enlarged bottom perspective view thereof; and
Fig. 16 is an enlarged cross-sectional elevational view of a portion of the protector support inside a needle protector according to the second preferred embodiment of the present invention.

### DETAILED DESCRIPTION OF THE INVENTION

Certain terminology is used in the following description for convenience only and is not limiting. The words "bottom" and "top" designate directions in the drawings to which reference is made. Hereinafter, the terms "proximal" and "rear" are synonyms, as are the terms "distal" and "front." The word "inwardly" and "outwardly" refer to directions toward and away from, respectively, the geometric center of the device, and designated parts thereof, in accordance with the present invention. Unless specifically set forth herein, the terms "a," "an" and "the" are not limited to one element, but instead should be read as meaning "at least one." The terminology includes the words noted above, derivatives thereof and words of similar import.

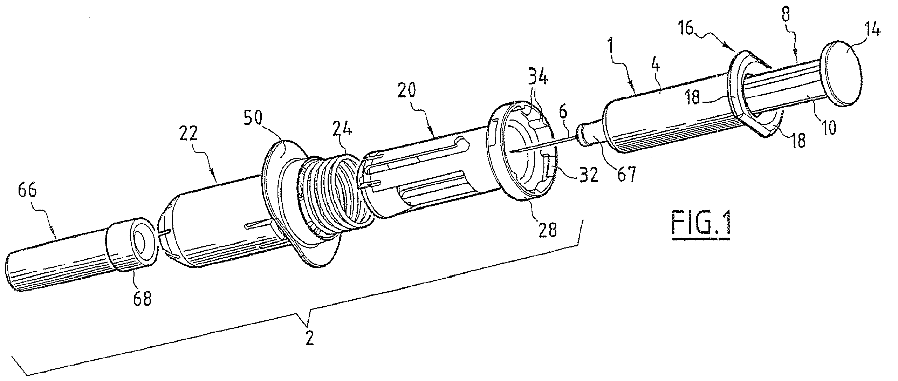

Referring to the drawings in detail, wherein like numerals indicate like elements throughout the several views, Figs. 1-13 show a needle protection assembly 2 for a syringe 1 according to a first preferred embodiment of the present invention. The syringe 1 is preferably a glass syringe of standard type, which is intended for single use, but the syringe 1 is not limited to such a type or functionality. The syringe 1 preferably contains a liquid to be injected into a patient in an intramuscular or subcutaneous manner. The syringe 1 preferably includes a body 4, a needle 6 which is fixedly joined to a distal end of the body 4, and a piston 8. The piston 8 preferably includes a rod 10 which is provided with a push-button 12 at the distal end thereof and a contact head 14, on which the thumb of the hand of the practitioner is intended to press.

The body 4 of the syringe 1 preferably includes a neck 16 at a proximal end thereof. The neck 16 preferably circumferentially delimits two diametrically opposed lugs 18 which are generally intended, in particular in the absence of the assembly 2, to form contact surfaces for the index finger and second finger of the practitioner during the manipulation of the syringe and the injection of the liquid therein.

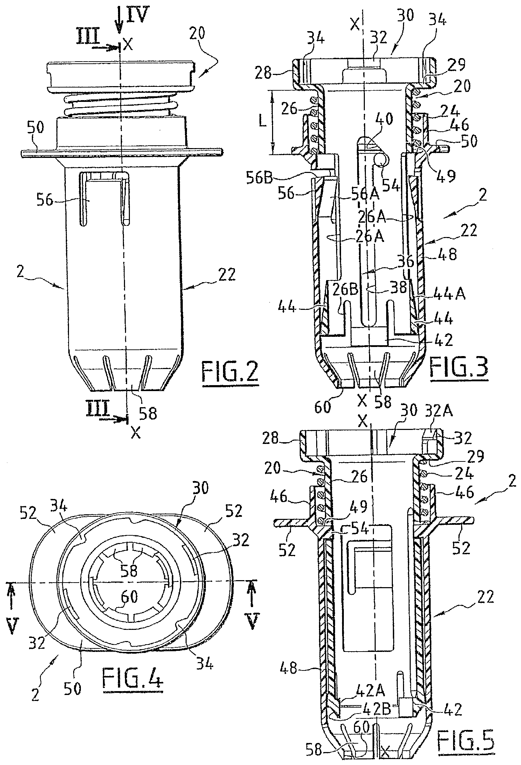

As shown in Fig. 2, the protection assembly 2 preferably generally includes an axis X-X. The protection assembly 2 also preferably includes a protector support 20 of generally tubular shape, a protective sleeve or needle protector 22 which is arranged coaxially relative to the support 20 and which has a diameter greater than that of the support, and a spring 24. The support 20 preferably includes a main portion 26 which is substantially cylindrical and which has an inside diameter substantially equal to the outside diameter of the body 4 of the syringe 1. The main portion 26 preferably continues at the proximal end thereof with a secondary portion 28 having larger inside and outside diameters than those of the main portion 26. A radial shoulder 29 is preferably formed at the proximal end of the main portion 26.

The proximal portion 28 of the protection assembly 2 is preferably provided with means 30 for fixing the protection assembly 2 to the syringe neck 16. In greater detail, these means 30 preferably include at least one and preferably a pair of diametrically opposed deformable hooks 32. Each hook 32 preferably has a substantially frustoconical inclined surface 32A which widens towards a free end of the secondary portion 28 and which is intended to be resiliently repelled by the lugs 18 of the syringe neck 16 when the support 20 is fixed to the syringe 1. The hooks 32 are remote from the shoulder 29 by a distance substantially equal to the thickness of the lugs 18. The hooks 32 thus form a clip-fit means for the syringe neck 16.

Referring to Figs. 1, 4 and 6B, the fixing means 30 preferably include at least one and preferably a plurality of protrusions 34 which project radially toward an interior of the secondary portion 28 of the protection assembly 2. The protrusions 30 are suitable for forming angular indexing means, at approximately 180°, of the neck 16 of the syringe 1 and rotation locking stops for the neck 16 when the neck 16 is axially retained between the hooks 32 and the shoulder 29.

Referring to Figs. 3, 9 and 13, at least one and preferably two traversing grooves 36 are arranged facing each other in the main portion 26 of the protection assembly 2. Each traversing groove 36 preferably includes a first rectilinear portion 38, which extends substantially along axis X-X of the support 20 over a length greater than that of the needle 6, and a second rectilinear portion 40 which extends in an inclined manner relative to the same axis X-X. The second rectilinear portion 40 opens at the proximal end portion of the first rectilinear portion 38 and preferably forms a V-shape, the point of which is directed towards the proximal side of the protection assembly 2.

As shown in Fig. 5, the main portion 26 of the protection assembly 2 preferably includes a first pair of diametrically opposed tongues 42 at the distal end thereof, which are each located in the continuation of the traversing grooves 36. The tongues 42 have an inner face 42A of substantially cylindrical shape and a substantially frustoconical outer face 42B which is divergent towards the rear.

As shown in Fig. 3, the main portion 26 of the protection assembly 2 also preferably includes a second pair of diametrically opposed tongues 44 which are located between the first tongues 42 along the circumference of the distal end of this portion. The second pair of tongues 44 have a substantially frustoconical outer surface 44A, which is divergent towards the front, and a substantially planar distal surface 44B. Plumb with the second tongues 44 at the proximal end, longitudinal recesses 26A are preferably arranged in the main portion 26. In addition, at one side and the other of the first tongues 42, axial slots 26B are provided starting from the distal end of the support in such a manner that, before the body 4 of the syringe 1 is inserted inside the support 20, the first tongues 42 can be deformed radially, in particular inwardly.

The protective sleeve 22 preferably has a length substantially equal to that of the body 4 of the syringe 1. The protective sleeve 22 preferably includes two cylindrical portions 46, 48. The proximal portion 46 of the protective sleeve 22 preferably includes a diameter that is at least slightly greater than that of the main portion 48 of the protective sleeve 22. The two portions 46, 48 of the protective sleeve 22 preferably fit together to form a radial shoulder 49. As shown in Fig. 4, the protective sleeve 22 preferably includes an external flange 50 in the proximal portion thereof in the form of two diametrically opposed lugs 52.

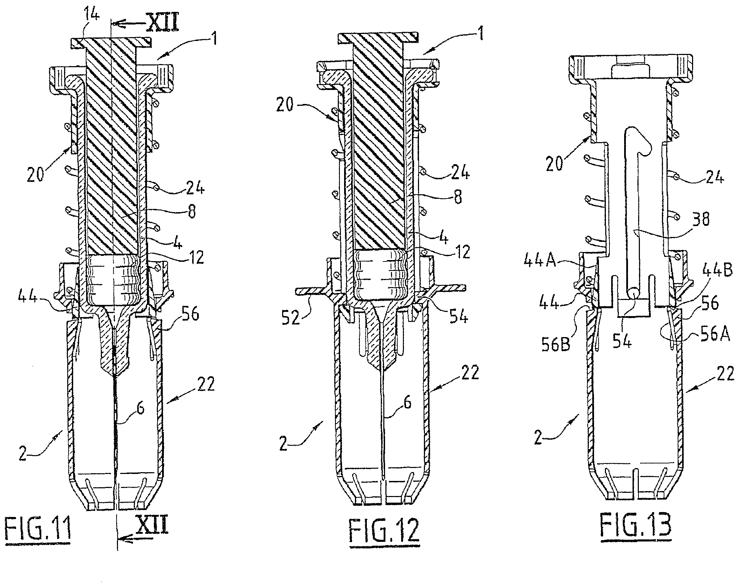

Referring to Fig. 5, at least one and preferably two diametrically opposed studs 54 are preferably fixedly joined to the protective sleeve 22 in the proximal portion 46 and extend radially inwardly therefrom. The two studs 54 are suitable for being received and guided in the two respective traversing grooves 36 of the support 20. The support 20 and the sleeve 22 are thus movable relative to each other in translation along the common axis thereof and with limited rotation about the same axis when each stud 54 is located in the respective second rectilinear portion 40. More specifically, the support 20 and the sleeve 22 can be moved between a retracted position (see Figs. 2-7), in which the greater part of the sleeve 22 covers the greater part of the support 20 and the studs 54 are located at the distal end of each of the second rectilinear portions 40, and a deployed position (see Figs. 11-13), in which the sleeve 22 projects axially from the support 20 and the studs 54 are located at the distal end of the longitudinal groove portion 38. When the syringe 1 is fixed to the assembly 2, the above-identified extreme positions correspond to an injection configuration, in which the needle 6 of the syringe 1 is disengaged and intended to be inserted into a patient, and a protection configuration, in which this needle is surrounded by the protective sleeve 22, respectively.

Referring to Fig. 3, the proximal portion 28 of the sleeve 22 preferably includes a pair of diametrically opposed deformable hooks 56 that extend radially inwardly therefrom. Each of the hooks 56 has an inner surface 56A of substantially frustoconical shape which is divergent towards the front and which is complementary to that of the inner surfaces 44A of the tongues 44 of the support 20, and a proximal surface 56B which is substantially planar. In the retracted position of the sleeve 22, the hooks 56 extend inside the recesses 26A which are arranged in the support 20. In the deployed position of the sleeve 22, faces 56B of the hooks 56 are stopped axially against the second tongues 44 (see Fig. 11). As a result, the hooks 56 and the second tongues 44 in this manner form a rigidly locking assembly in the deployed position.

Referring to Figs. 3 and 5, the sleeve 22 is preferably further provided at the distal end thereof with a crown of deformable tongues 58. Distal edges of the tongues 58 preferably form a substantially circular opening 60 having a diameter less than the inside diameter of the main portion 26 of the support 20.

As shown in Figs. 3, 5, 6A and 7-13, a spring 24, which is preferably a helical spring, is preferably arranged between the protective sleeve 22 and the protector support 20. More specifically, the spring 24 is received between the shoulder 29 of the support 20 and the shoulder 49 of the sleeve 22. In the retracted position of the sleeve 22, the spring 24 is in a compressed state, thus having decompression energy linked to the tension of the spring 24 and the difference between the length of the spring 24 in the rest state and its length, designated L in Figs. 2 to 7, in the compressed state. In other words, the spring 24 has a predetermined supplementary compression force threshold which corresponds to the minimum force necessary to further compress the spring 24 from the initial compressed state shown in Figs. 2-7. The tension of the spring 24 and/or initial compression length L are selected so that this predetermined force threshold is greater than the pushing effort necessary to displace the piston 8 of the syringe 1 over the entire injection path thereof. More specifically, the predetermined force of the spring 24 in the locked state is greater than the sum of the injection effort. The injection effort is defined herein as the effort for evacuating the liquid out of the needle 6 of the syringe 1, and the efforts for detaching and sliding the push-button 12 inside the body 4 of the syringe 1.

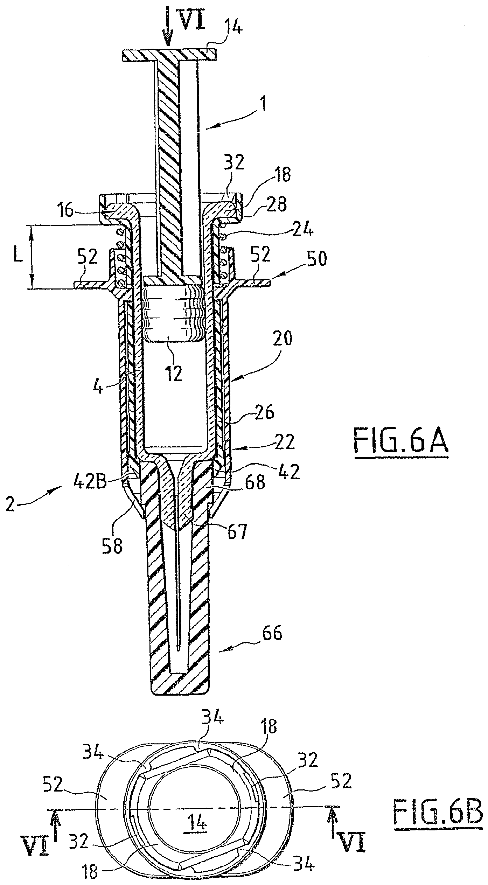

As shown in Figs. 1 and 6A, the protection assembly 2 may include a cap 66 of generally tubular shape. The cap 66 is suitable for surrounding the needle 6 before the syringe 1 is used. The cap 66 is preferably closed at one of the ends thereof. The opposite end of the cap 66 is preferably formed by an annular ring 68 having an outside diameter suitable both for being conjugate with the surface 42A of the hooks 42 of the support 20 and for being greater than the diameter of the opening 60 formed by the tongues 58 of the protective sleeve 22. The inner face of the ring 68 is intended to be adhesively bonded to the glass base 67 of the syringe body 4, where the needle 6 is fixed, in particular to ensure given sealing with respect to bacteria.

The operation of the injection device according to the invention is as follows:

Initially, it is preferred that the protection device 2 is assembled in the retracted configuration (see Figs. 2-7). The protective sleeve 22 is then preferably fitted around the support 20, starting from the distal end of the support 20, with the spring 24 being arranged therebetween. More specifically, the sleeve 22 is displaced axially backwards relative to the support 20, while at the same time radially deforming the hooks 56 outwards by means of a suitable tool, at least until the hooks 56 axially reach the front portion of the longitudinal recesses 26A. With the sleeve 22 still being displaced backwards, the studs 54 are then pressed against the outer surfaces 42B of the tongues 42, with the tongues 42 being deformed inwardly, until the studs 54 are received in the groove portions 38. The displacement of the protector 22 backwards then continues until the studs 54 are received in the second rectilinear portions 40, the support 20 and the protector 22 being pivoted relative to each other. The protector 22 is thereby located in the retracted position. The glass syringe 1 is preferably pre-filled with a liquid to be injected into a patient. The syringe 1 is provided with the cap 66 which encloses the base 67 of the syringe body 4

The syringe 1 and the cap 66 are then preferably inserted inside the assembly 2 in order to form the injection device, as illustrated in Figs. 6A and 6B. More specifically, the body 4 of the syringe 1 is displaced substantially axially inside the support 20 until the syringe neck 16, which is indexed by the protrusions 34, clips inside the proximal portion 28, the hooks 32 being deformed radially outwards. The lugs 18 of the syringe neck 16 are then preferably retained axially by the hooks 32, while the peripheral protrusions 34 retain the syringe neck 16, and therefore the syringe 1, in terms of rotation with respect to the support 20.

Further, insofar as the syringe neck 16 is completely clipped inside the portion 28, the neck 16 can no longer serve as usual to form a contact surface for the index finger and the second finger 9 of the practitioner. This support function is fulfilled by the flange 50 which is fixedly joined to the sleeve 22. Insofar as the length of the protective sleeve 22 is substantially equal to that of the syringe body 4 and/or the flange 50 is arranged in the region of the proximal end of the sleeve 22, the practitioner can manipulate the syringe 1 by resting his/her thumb on the contact head 14 of the piston 8 as in the accustomed manner, and by resting his index finger and second finger on the faces of the lugs 52 that are directed towards the needle 6. Furthermore, when the syringe 1 is fixed to the protector support 20, as illustrated in Figs. 6A and 6B, the cap 66 preferably projects outside the protective sleeve 22.

When the practitioner is ready to carry out the injection of the liquid contained in the syringe, he/she retracts the cap 66 by drawing it axially forward. The ring 68 then passes through the opening 60, deforming the tongues 58. Once the cap 66 has been retracted, the tongues 58 again take up the initial position thereof. The passage hole 60 at the distal end of the sleeve 22, having a diameter smaller than the outside diameter of the cap 66, then preferably prevents the cap 66 from being repositioned around the needle. The co-operation of the cap 66 and the tongues 58 thus forms a control means for the first use of the injection device.

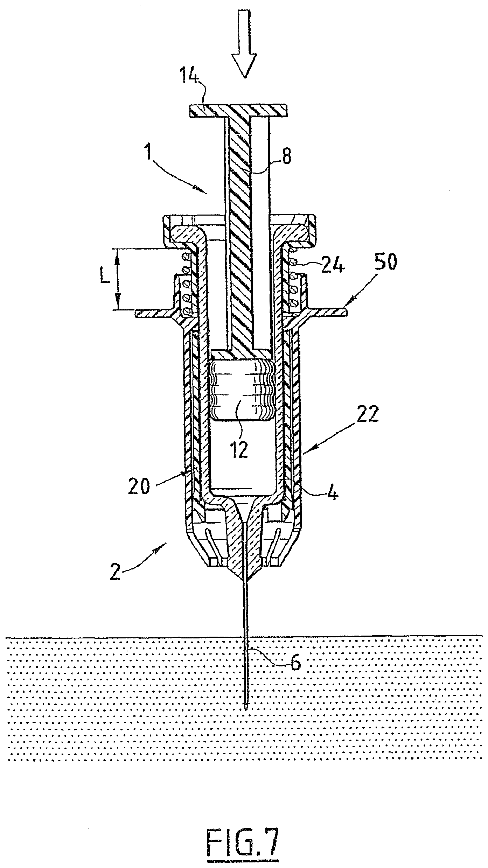

The practitioner then inserts the needle 6 into a patient. He/she injects the liquid contained in the syringe 1 by applying a pushing action to the contact head 14 of the piston 8, his/her index finger and second finger remaining in contact with the faces of the lugs 52 that are directed towards the needle. During the injection, no movement is brought about between the protector support 20 and the protective sleeve 22, the spring 24 remaining compressed with a length L, as illustrated in Fig. 7. The injection continues until the push-button 12 of the piston 8 reaches the end of the injection path.

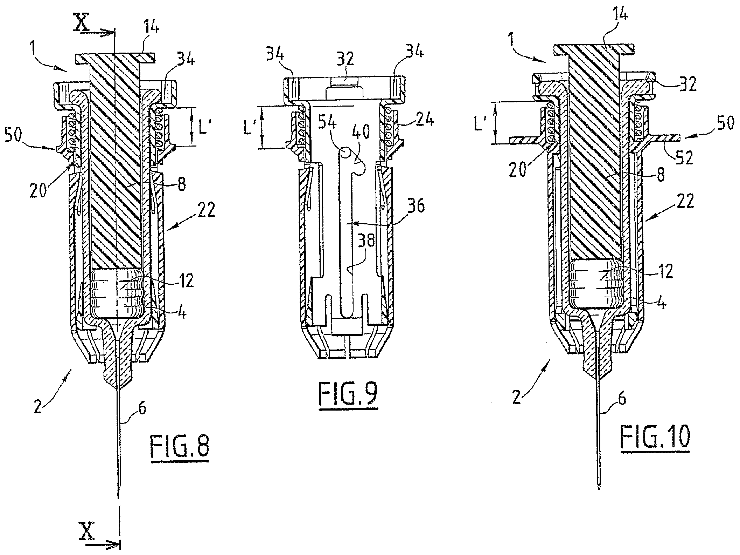

The practitioner then withdraws the needle from the patient. In order to release the protection assembly 2, the practitioner preferably applies an additional pressure to the piston rod 8. This pressure must be greater than the predetermined force produced by the spring 24 in the locked state, so that the spring 24 is further compressed and changes from the length L thereof to a smaller length L', as illustrated in Figs. 8 to 10. To this end, considering the support 20 to be stationary, the protective sleeve 22 is displaced axially toward the proximal end of the support 20. The practitioner brings about this movement by applying a corresponding pressure by means of his index finger and his second finger to the lugs 52 of the flange 50 of the sleeve 22. This pressure brings about, in a manner combined with the translation movement, the rotation of the protective sleeve 22 about the syringe support 20, the studs 54 being guided by the second rectilinear portions 40, respectively. This rotation movement continues until the studs reach the proximal end of the respective second rectilinear portion 40, that is to say, the proximal end of the longitudinal groove portion 38, as shown in Fig. 9. The device 2 is then in the unlocking position of the spring 24.

The practitioner then releases the pressure which he/she had been applying to the flange 50 until then, allowing the spring 24 to relax as far as a rest state. The studs 54 preferably move in translation inside the longitudinal groove portion 38 as far as the distal end thereof, as illustrated in Fig. 13. The translation movement of the sleeve 22 relative to the support 20 can be controlled by the practitioner if he/she progressively releases the finger-hold which he/she applies to the flange 50. When the studs 54 have arrived at the distal end of the respective traversing groove 36 (Fig. 13), the protector is in the deployed position thereof.

Furthermore, when the sleeve 22 is moving in translation relative to the support 20, the hooks 56 make use of the longitudinal recesses 26A of the support 20 until they slide along the distal tongues 44 of the support 22 by co-operation between the complementary surfaces 56A and 44A thereof.

In the deployed position of the protector 22, the tongues 44 retain the hooks 56 in position by co-operation between the surfaces 56B, 44B so that the protective sleeve 22 cannot be moved into the initial position thereof. Similarly, the sleeve 44 cannot readily be detached from the support 20 because the studs 54 are stopped against the distal base of the longitudinal groove portion 38 (FIG. 13), the tongues 42 forming this base being radially retained between the body of the syringe 1 and the sleeve 22

The injection device according to the present invention is simple to use in this manner, while at the same time allowing the practitioner to control the movement for covering the needle with the protective sleeve 22. The number of components constituting the protection assembly 2 illustrated is reduced to three. The device according to the present invention can be adapted to various types of syringe, both in terms of the shapes and the volumes thereof. Therefore, the device has the advantage of not calling into question the general shape of the syringes used and consequently does not bring about any modification of the industrial methods for filling these syringes.

Various variants of the device according to the present invention can be envisaged: unlike the embodiment described above, the studs 54 and/or the flange 50 of the protective sleeve 22 can be separate from the sleeve 22 and not produced integrally therewith; contrary to the device described, the studs 54 can be produced on the outer surface of the protector support 20 and the traversing groove 36 arranged in the protective sleeve 22; the support 20 can be produced in one piece with the syringe body 4; and/or the protrusions 34 of the means 30 for fixing the support 20 to the syringe neck 16 can be dispensed with and optionally replaced with one or more hooks which are similar to the hooks 32 described above, the assembly of these hooks axially retaining the neck 16 relative to the support 20 for any relative angular position between the syringe 1 and the support 20; in this case, the syringe 1 is free to rotate inside the support 20 which, on the other hand, ensures greater ease of fixing the protection assembly 2 to the syringe (absence of indexing).

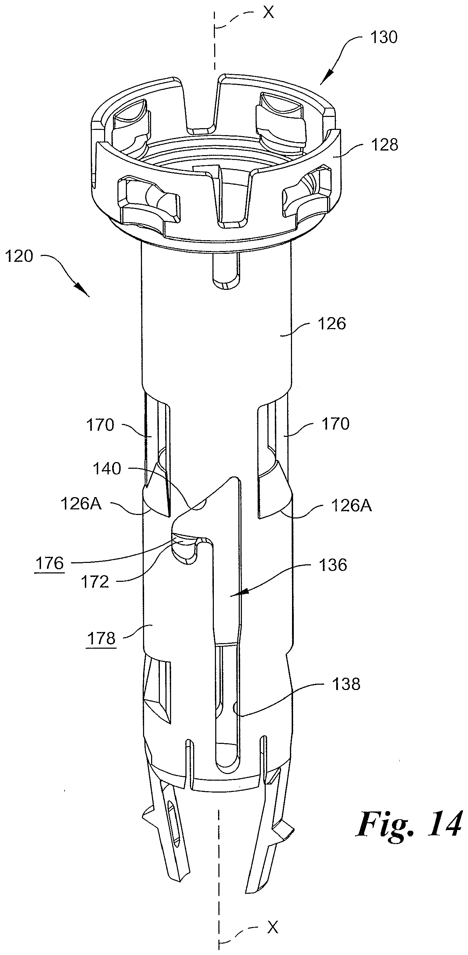

Figs. 14-16 show a second preferred embodiment of a protector support 120 in combination with other features of the present invention. The reference numerals of the second preferred embodiment are distinguishable from those of the first preferred embodiment by an addition of one-hundred (100), but otherwise indicate the same or similar elements as indicated in the first preferred embodiment, except as otherwise specified. At least certain portions of the support 120 of the second preferred embodiment are substantially similar to those of the first preferred embodiment described above. The description of certain similarities between the embodiments may be omitted herein for the sake of brevity and convenience, and, therefore, is not limiting. The syringe 1 and needle protector 22 described above are used in a substantially similar manner with the support 120 of the second preferred embodiment as they are used with the support 20 of the first preferred embodiment, except as otherwise indicated below.

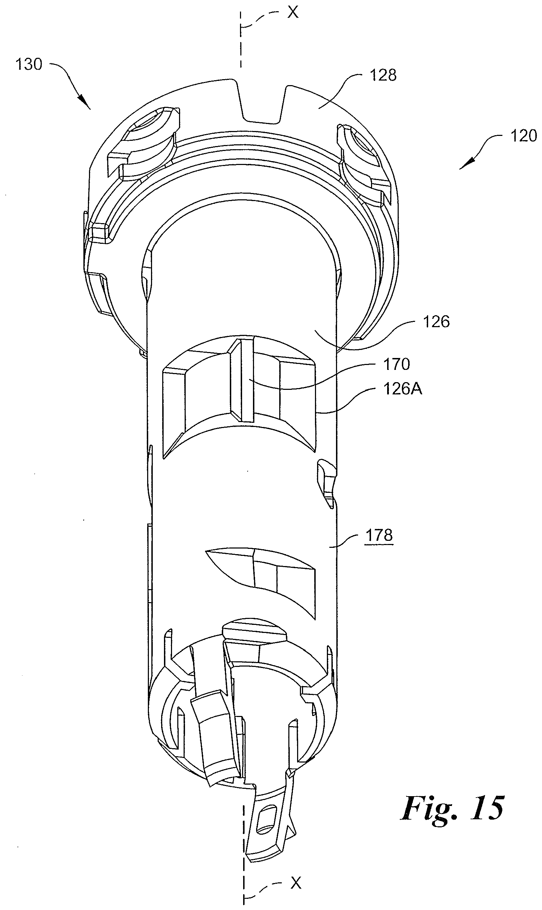

Referring to Figs. 14 and 15, a distinguishing feature of the second preferred embodiment is that at least one rib 170 preferably extends axially across at least a portion of each recess 126A in the main portion 126 of the support 120. Each of the two diametrically opposed recesses 126A preferably extend completely through at least a portion of the main portion 126 of the support 120. More preferably, one generally rigid rib 170 preferably extends axially across an entire height of each recess 126A. In other words, it is preferred that the rib 170 extends from one point on or around the respective recess 126A to an opposing point on or around the respective recess 126A. As shown in Fig. 15, each rib 170 is preferably at least generally centrally positioned (circumferentially) with the respective recess 126A and preferably has a radially thickness that is at least slightly less than that of the support 120. As shown in Figs. 14 and 15, each rib 170 preferably extends at least generally parallel to the axis X-X of the device. Each rib 170 may have generally sharp edge or corners, such that abutting walls or surfaces of each rib 170 form an approximately 90° angle (see Figs. 14 and 15). Alternatively, one or more edges or corners of each rib 170 may be at least slightly arcuate, curved and/or chamfered, for example, so as to reduce the force required for an object, such as one of the hooks 56, to pass thereover.

In operation, when the needle protector 22 is rotated with respect to the protector support 120, at least one of the hooks 56 (see Fig. 3) of the needle protector 22 preferably contacts and snaps over at least one of the ribs 170, which preferably locks the rotational position of the support 120 with respect to the needle protector 22. The contact between and eventually snapping of the hook 56 over the rib 170 preferably produces a tactile or audible indication (click) to a user to indicate that such functionality has occurred. More preferably, each of the two diametrically opposed hook 56 contacts and snaps over one of the two ribs 170 when the needle protector 22 is sufficiently rotated with respect to the support 120. The tactile or audible indication preferably confirms that the safety feature has been properly and irreversibly activated and that the device is ready for use.

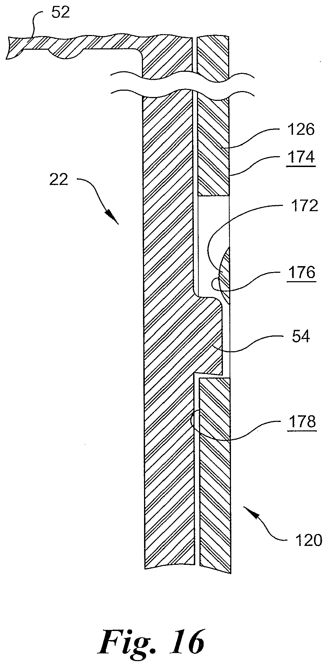

Referring to Figs. 14 and 16, another distinguishing feature of the second preferred embodiment is the inclusion of at least one and preferably two diametrically opposed actuation bumps 172 on the protector support 120. At least a portion of each actuation bump 172 is preferably arcuate or convex and extends radially outwardly beyond an interior surface 174 (see Fig. 16) of the support 120. It is preferred that an exterior surface 176 of each actuation bump 172 is positioned at least slightly radially inwardly from an exterior surface 178 of the support 120, but the present invention is not limited to such a configuration. Each actuation bump 172 is preferably generally rigid and positioned within and/or extends across at least a portion one of the two diametrically opposed traversing grooves 136 formed in the support 120. More specifically, it is preferred that each actuation bump 172 is positioned proximate to one end, such as a distal end, of the second rectilinear portion 140 of one of the traversing grooves 136. The specific size, shape, configuration, orientation and/or material used to form the ribs 170 and/or the actuation bumps 172 may be modified as desired to optimize actuation of the device and/or reduce the activation spring force. The reduction of the activation spring force may lower the cost of the spring 24 and provide the added advantage of lowering the overall stress load imposed on the polymeric components of the needle-protection device 2 during assembly and long-term storage.

Operation of the second preferred embodiment is described below with respect to a single stud 54 and respective actuation bump 172, as well as a single hook 56 and respective rib 170. However, the below method of operation is equally applicable with respect to two or more of each of the above-identified structures. Upon completion of an injection, the user or healthcare professional preferably applies sufficient pressure or force to the contact head 14 of the syringe 1 to compress the spring 24. With references to Figs. 14 and 16, once the requisite force is applied, the needle protector 22 moves or traverses upwardly with respect to the support 120 and at least one of the studs 54 contacts or engages at least a portion of one of the actuation bumps 172. With continued force applied by the user, the stud 54 rides or snaps over the respective actuation bump 172 and eventually moves past or disengages from the actuation bump 172. The momentum produced from the above causes the stud 54 to move into contact or engagement with at least a portion of the traversing groove 136, such as the second linear portion 140 thereof. Engagement between the stud 54 and the second linear portion 140 causes the protector 22 to cam in rotation with respect to the support 120. As the protector 22 continues to rotate with respect to the support 120 such that the stud 54 approaches and/or contacts the proximal or upper end of the second linear portion 140 of the traversing groove 136, at least one of the hooks 56 of the protector 22 rides or snaps over one of the ribs 170, which preferably locks the rotational position of the support 120 and the protector 22. In this configuration, the stud 54 is preferably axially aligned with the first linear portion 138 of the traversing groove 136 for full deployment of the protector 22 and the stud 54 is not able to re-enter the "J" shaped area of the traversing groove 136 defined by the second linear portion 140 because the engagement of the hook 56 and the recess 126A prevents such movement. Thus, the stud 54 is preferably positioned within the first rectilinear portion 138 of the traversing groove 136 when the hook snaps over the rib 170 to permit the stud 54 to slide distally along the axially-extending traversing groove 136 for extending the needle protector 22 to cover the needle 6 following injection.

It will be appreciated by those skilled in the art that changes could be made to the embodiments described above without departing from the broad inventive concept thereof. It is understood, therefore, that this invention is not limited to the particular embodiments disclosed, but it is intended to cover modifications within the scope of the present invention as defined by the appended claims.

## Claims

1. A needle-protection device (2) for a syringe (1), the device comprising:
a protector support (120) having a main portion (126) and a secondary portion (128), the main portion having a generally tubular shape and including at least one recess (126A) extending therethrough and at least one rib (170) extending across at least a portion of the recess, the rib being generally centrally positioned within the recess, the secondary portion includes at least one projection (34) or groove capable of fixing the protector support to a neck (16) of the syringe; and
a needle protector (22) having a generally tubular shape, an inner diameter of the needle protector being at least slightly greater than an outer diameter of the protector support such that at least a portion of the protector support can be inserted within at least a portion of the needle protector, at least one hook (56) extending radially inwardly from the needle protector,
wherein the needle protector is rotatable with respect to the protector support such that the at least one hook (56) snaps over the at least one rib and locks the rotational position of the protector support with respect to the needle protector.

2. The device according to claim 1, wherein the device extends along an axis (X-X) and the rib extends generally parallel to the axis.

3. The device according to claim 1, wherein the recess extends completely through at least a portion of the main portion of the protector support.

4. The device according to claim 1, wherein movement of the hook over the rib produces a tactile or audible indication.

5. The device according to claim 1, further comprising:
two diametrically opposed recesses (126A) extending through the main portion of the protector support;
a rib (170) extending across at least a portion of each recess; and
two diametrically opposed hooks (56) extending radially inwardly from the needle protector.

6. The device according to claim 1, further comprising:
at least one traversing groove (136) extending through the main portion of the protector support;
an actuation bump (172) on the protector support; and
at least one stud (54) extending radially inwardly from the needle protector,
wherein the stud contacts and moves over the actuation bump when the needle protector is rotated with respect to the protector support.

7. The device according to claim 6, wherein the actuation bump is arcuate.

8. The device according to claim 6, wherein at least a portion of the actuation bump extends radially outwardly beyond an interior surface of the protector support.

9. The device according to claim 6, wherein the actuation bump is positioned within at least a portion of the traversing groove.

10. The device according to claim 6, wherein the traversing groove includes a first rectilinear portion (138) extending substantially along an axis (X-X) of the protector support and a second rectilinear portion (140) extending in an inclined manner with respect to the axis.

11. The device according to claim 10, wherein at least a portion of the actuation bump is positioned proximate to one end of the second rectilinear portion of the traversing groove.

12. The device according to claim 11, wherein the stud (54) is positioned within the first rectilinear portion (138) of the traversing groove (136) when the hook (56) snaps over the rib (170).

13. The device according to claim 1, further comprising:
two diametrically opposed traversing grooves (136) extending through the main portion of the protector support;
two diametrically opposed actuation bumps (172) on the protector support; and
two diametrically opposed studs (54) extending radially inwardly from the needle protector, wherein each stud contacts one of the actuation bumps when the needle protector is rotated with respect to the protector support.

14. The device according to claim 1, wherein the rib extends across the entire recess.

## Patentansprüche

1. Nadelschutzvorrichtung (2) für eine Spritze (1), wobei die Vorrichtung umfasst:
einen Schutzträger (120) mit einem Hauptbereich (126) und einem Sekundärbereich (128), wobei der Hauptbereich eine allgemeine Rohrform aufweist und mindestens eine Ausnehmung (126A) hat, die sich durch ihn hindurch erstreckt, und mindestens eine Rippe (170), die sich zumindest über einen Teil der Ausnehmung erstreckt, wobei die Rippe allgemein zentral in der Ausnehmung positioniert ist, wobei der Sekundärbereich mindestens einen Vorsprung (34) oder eine Nut aufweist, der/die zum Festlegen des Schutzträgers an einem Hals (16) der Spritze ausgebildet ist; und
einen Nadelschutz (22), der eine allgemeine Rohrform aufweist, wobei ein Innendurchmesser des Nadelschutzes zumindest geringfügig größer ist als ein Außendurchmesser des Schutzträgers, so dass zumindest ein Teil des Schutzträgers in zumindest einen Teil des Nadelschutzes eingeführt werden kann, wobei sich zumindest ein Haken (56) von dem Nadelschutz radial nach innen erstreckt,
wobei der Nadelschutz bezüglich des Schutzträgers derart drehbar ist, dass der wenigstens eine Haken (56) über die wenigstens eine Rippe schnappt und die Drehposition des Schutzträgers bezüglich des Nadelschutzes sichert.

2. Vorrichtung nach Anspruch 1, wobei sich die Vorrichtung entlang einer Achse (X-X) und die Rippe allgemein parallel zu der Achse erstreckt.

3. Vorrichtung nach Anspruch 1, wobei sich die Ausnehmung vollständig durch mindestens einen Teil des Hauptbereichs des Schutzträgers erstreckt.

4. Vorrichtung nach Anspruch 1, wobei die Bewegung des Hakens über die Rippe einen fühlbaren oder hörbaren Hinweis erzeugt.

5. Vorrichtung nach Anspruch 1, ferner umfassend:
zwei einander diametral gegenüberliegende Ausnehmungen (126A), die sich durch den Hauptbereich des Schutzträgers erstrecken;
eine Rippe (170), die sich über mindestens einen Teil jeder Ausnehmung erstreckt; und
zwei einander diametral gegenüberliegende Haken (56), die sich von dem Nadelschutz radial nach innen erstrecken.

6. Vorrichtung nach Anspruch 1, ferner umfassend:
zumindest eine durchlaufende Nut (136), die sich durch den Hauptbereich des Schutzträgers erstreckt;
einen Betätigungshöcker (172) an dem Schutzträger; und
mindestens einen Zapfen (54), der sich von dem Nadelschutz radial nach innen erstreckt,
wobei der Zapfen den Betätigungshöcker kontaktiert und sich über den Betätigungshöcker bewegt, wenn der Nadelschutz bezüglich des Schutzträgers gedreht wird.

7. Vorrichtung nach Anspruch 6, wobei der Betätigungshöcker bogenförmig ist.

8. Vorrichtung nach Anspruch 6, wobei sich zumindest ein Teil des Betätigungshöckers radial nach außen über eine Innenfläche des Schutzträgers hinaus erstreckt.

9. Vorrichtung nach Anspruch 6, wobei der Betätigungshöcker zumindest in einem Teil der durchlaufenden Nut positioniert ist.

10. Vorrichtung nach Anspruch 6, wobei die durchlaufende Nut einen ersten geradlinigen Abschnitt (138) aufweist, der sich im Wesentlichen entlang einer Achse (X-X) des Schutzträger erstreckt, und einen zweiten geradlinigen Abschnitt (140), der sich bezüglich der Achse geneigt erstreckt.

11. Vorrichtung nach Anspruch 10, wobei zumindest ein Teil des Betätigungshöckers in der Nähe eines Endes des zweiten geradlinigen Abschnitts der durchlaufenden Nut positioniert ist.

12. Vorrichtung nach Anspruch 11, wobei der Zapfen (54) in dem ersten geradlinigen Abschnitt (138) der durchlaufenden Nut (136) positioniert wird, wenn der Haken (56) über der Rippe (170) einrastet.

13. Vorrichtung nach Anspruch 1, ferner umfassend:
zwei einander diametral gegenüberliegende durchlaufende Nuten (136), die sich durch den Hauptbereich des Schutzträgers erstrecken;
zwei einander diametral gegenüberliegende Betätigungshöcker (172) an dem Schutzträger; und
zwei einander diametral gegenüberliegende Zapfen (54), die sich von dem Nadelschutz radial nach innen erstrecken,
wobei jeder Zapfen einen der Betätigungshöcker kontaktiert, wenn der Nadelschutz bezüglich des Schutzträgers gedreht wird.

14. Vorrichtung nach Anspruch 1, wobei sich die Rippe über die gesamte Ausnehmung erstreckt.

## Revendications

1. Dispositif de protection d'aiguille (2) pour une seringue (1), le dispositif comprenant:
un support de protecteur (120) ayant une partie principale (126) et une partie secondaire (128), la partie principale présentant une forme généralement tubulaire et comportant au moins un évidement (126A) s'étendant à travers celle-ci, et au moins une nervure (170) traversant au moins une partie de l'évidement, ladite nervure étant généralement positionnée au centre dans l'évidement, la partie secondaire comportant au moins une saillie (34) ou rainure capable de fixer le support de protecteur sur cou (16) de la seringue; et
un protecteur d'aiguille (22) présentant une forme généralement tubulaire, un diamètre intérieur du protecteur d'aiguille étant légèrement supérieur à un diamètre extérieur du support de protecteur de telle sorte qu'au moins une partie du support de protecteur peut être insérée dans au moins une partie du protecteur d'aiguille, au moins un crochet (56) s'étendant radialement vers l'intérieur depuis le protecteur d'aiguille,
dans lequel le protecteur d'aiguille est rotatif par rapport au support de protecteur de telle sorte que ledit au moins un crochet (56) s'encliquette sur ladite au moins une nervure et verrouille la position de rotation du support de protecteur par rapport au protecteur d'aiguille.

2. Dispositif selon la revendication 1, dans lequel celui-ci s'étend le long un axe (X-X) et la nervure s'étend généralement parallèlement audit axe.

3. Dispositif selon la revendication 1, dans lequel l'évidemment s'étend complètement à travers au moins une partie de la partie principale du support de protecteur.

4. Dispositif selon la revendication 1, dans lequel le mouvement du crochet sur la nervure produit une indication tactile ou audible.

5. Dispositif selon la revendication 1, comprenant en outre:
deux évidements (126A) diamétralement opposés l'un de l'autre et s'étendant à travers la partie principale du support de protecteur;
une nervure (170) traversant au moins une partie de chaque évidemment; et
deux crochets (56) diamétralement opposés l'un de l'autre et s'étendant radialement vers l'intérieur depuis le protecteur d'aiguille.

6. Dispositif selon la revendication 1, comprenant en outre:
au moins une rainure traversante (136) s'étendant à travers la partie principale du protecteur de support;
une bosse d'actionnement (172) sur le support de protecteur; et
au moins un goujon (54) s'étendant radialement vers l'intérieur depuis le protecteur d'aiguille,
dans lequel le goujon entre en contact avec la bosse d'actionnement et passe au-dessus de celle-ci lorsque le protecteur d'aiguille est tourné par rapport au support de protecteur.

7. Dispositif selon la revendication 6, dans lequel la bosse d'actionnement est arquée.

8. Dispositif selon la revendication 6, dans lequel au moins une partie de la bosse d'actionnement s'étend radialement vers l'extérieur au-delà d'une surface intérieur du support de protecteur.

9. Dispositif selon la revendication 6, dans lequel la bosse d'actionnement est positionnée dans au moins une partie de la rainure traversante.

10. Dispositif selon la revendication 6, dans lequel la rainure traversante comporte une première partie rectilinéaire (138) s'étendant sensiblement le long d'un axe (X-X) du support de protecteur, et une deuxième partie rectilinéaire (140) s'étendant de manière inclinée par rapport à l'axe.

11. Dispositif selon la revendication 10, dans lequel au moins une partie de la bosse d'actionnement est positionnée à proximité d'une extrémité de la deuxième partie rectilinéaire de la rainure traversante.

12. Dispositif selon la revendication 11, dans lequel le goujon (54) est positionné dans la première partie rectilinéaire (138) de la rainure traversante (136) lorsque le crochet (56) s'encliquette sur la nervure (170).

13. Dispositif selon la revendication 1, comprenant en outre:
deux rainures traversantes (136) diamétralement opposées l'une de l'autre et s'étendant à travers la partie principale du support de protecteur;
deux bosses d'actionnement (172) diamétralement opposées l'une de l'autre sur le support de protecteur; et
deux goujons (54) diamétralement opposés l'un de l'autre et s'étendant radialement vers l'intérieur depuis le protecteur d'aiguille,
dans lequel chaque goujon entre en contact avec une des bosses d'actionnement lorsque le protecteur d'aiguille est tourné par rapport au support de protecteur.

14. Dispositif selon la revendication 1, dans lequel la nervure s'étend à travers la totalité de l'évidement.
